# FASCICULE DE BREVET EUROPEEN

(11) **EP 1 565 179 B1**
(45) Date de publication et mention de la délivrance du brevet: **14.01.2009**
(21) Numéro de dépôt: 03789497.9
(22) Date de dépôt: 25.11.2003
(51) Int. Cl.: A61K 31/325, C07C 271/06, A61P 17/00

(54) **COMPOSITION COMPRENANT UN DERIVE D'ACIDE CARBAMIQUE, SON UTILISATION COSMETIQUE ET COMME MEDICAMENT**
ZUSAMMENSETZUNG ENTHALTEND EIN DERIVAT DER KARBAMINSÄURE, IHRE KOSMETISCHE VERWENDUNG IN DER KOSMETIK UND ALS ARZNEIMITTEL
COMPOSITION COMPRISING A CARBAMIC ACID DERIVATIVE, ITS USE AS COSMETIC AND MEDICINE

(30) Priorité: 25.11.2002 FR 0214792
(43) Date de publication de la demande: 24.08.2005
(73) Titulaire: Laboratoires Expanscience, 92400 Courbevoie (FR)
(72) Inventeur: PICCIRILLI, Antoine, F-78000 Versailles (FR); MSIKA, Philippe, F-78000 Versailles (FR); PICCARDI, Nathalie, F-38120 Saint Egrève (FR)
(74) Mandataire: Warcoin, Jacques
(86) Numéro de dépôt international: PCT/FR2003/003482
(87) Numéro de publication internationale: WO 2004/050079

(56) Documents cités:
- EP-A- 1 201 649
- WO-A-96/39385
- GB-A- 850 003
- US-A- 3 553 254
- US-A- 5 389 682
- US-A- 5 856 354

## Description

La présente invention se rapporte au traitement cosmétique et pharmaceutique, notamment dermatologique, de la peau et/ou des muqueuses. Plus particulièrement, la présente invention concerne une composition contenant au moins un dérivé hydroxylé ou alcoxylé d'acide carbamique, éventuellement en association avec au moins un autre composé tel qu'un inhibiteur de métalloprotéases, un inhibiteur de PKC, un agent anti-inflammatoire, un agent apaisant, un immunosuppresseur, un chélateur d'ions, une oxazoline et un alcanolamide. L'invention a également pour objet une telle composition pour son utilisation comme médicament, notamment pour son utilisation dans la prévention ou le traitement des pathologies cutanées d'origine allergique et/ou inflammatoire et/ou irritative ou de toute pathologie cutanée consécutive à la migration de cellules, telles que les cellules de Langerhans, induites par un signal efficace de migration, dit signal danger.

L'invention a également pour objet une méthode de traitement cosmétique des peaux et/ou des muqueuses sensibles, irritées, intolérantes, à tendance allergique, âgées, soumises à un signal danger, présentant un trouble de la barrière cutanée, présentant des rougeurs cutanées, ou présentant un trouble, un déséquilibre ou un désordre immunologique non pathologique, consistant à appliquer sur la peau et/ou les muqueuses une telle composition, consistant à appliquer sur la peau et/ou les muqueuses une telle composition.

Les dérivés d'acide carbamique selon la présente invention permettent d'inhiber la migration des cellules impliquées dans la réponse immunitaire et/ou inflammatoire et/ou irritative telles que les monocytes, les lymphocytes, les dendrocytes dermiques, les cellules dendritiques et plus particulièrement les cellules de Langerhans suite à un stimulus extérieur ou « signal danger » d'origine chimique, physique, biologique et plus particulièrement immunitaire, ou par suite au vieillissement intrinsèque (chronologique), hormonal ou extrinsèque (soleil, pollution), dont l'intensité serait suffisamment importante pour induire une perturbation de l'homéostasie cutanée.

Une des principales fonctions de la peau est la protection de l'organisme contre des agressions du milieu extérieur. Cette protection est assurée en grande partie grâce à la coopération de cellules présentes dans la peau qui sont capables, en présence d'un agent nocif, de générer une réponse inflammatoire et/ou immunitaire dirigée contre l'agent nocif. Il s'agit des cellules dendritiques, notamment des cellules de Langerhans (CL) de l'épiderme, des dendrocytes dermiques, des monocytes, des lymphocytes, des kératinocytes, des mastocytes, et des cellules endothéliales vasculaires.

Les CL sont des cellules dendritiques issues de la moelle épinière et qui résident dans les tissus non lymphoïdes tels que la peau et les muqueuses (bouche, poumon, vessie, rectum, vagin). Dans la peau, les CL s'intercalent entre les kératinocytes épidermiques en position suprabasale. Sur le plan ultrastructural, elles sont caractérisées par la présence d'un organite spécifique d'origine membranaire, le granule de Birbeck. Sur le plan immunohistochimique, les CL expriment notamment la molécule CD1a et les molécules du Complexe Majeur d'Histocompatibilité de classe II.

Les CL jouent un rôle déterminant dans l'immunité, en tant que cellules présentatrices de l'antigène. En effet, des expériences menées chez la souris démontrent que les CL capturent les antigènes présents au niveau de l'épiderme et migrent vers les tissus lymphoïdes drainant la peau, où elles présentent l'antigène aux cellules T. L'initiation de la réponse immune cutanée dépend de la capacité des CL à quitter l'épiderme pour migrer jusqu'aux ganglions proximaux. Différents facteurs peuvent influencer cette migration : l'expression de molécules d'adhérence, les protéines de la matrice extracellulaire, des haptènes, des cytokines, etc. Néanmoins, les mécanismes impliqués dans la migration des CL ne sont pas totalement encore élucidés. En particulier, avant d'atteindre les ganglions lymphatiques, les CL doivent non seulement traverser la jonction dermo-épidermique (JDE) mais également se frayer un chemin au travers de la matrice extracellulaire (MEC) dermique. La JDE est principalement composée de laminine 5, de collagènes de type IV et VII, de nidogène et de perlecan. La MEC qui entoure les fibroblastes du derme contient essentiellement des collagènes de type I et III.

La maturation, ainsi que l'initiation et la régulation de la migration des CL, sont sous la dépendance de cytokines pro-inflammatoires telles que l'IL-1β (interleukine-1-beta) et le TNF-α (Tumor Necrosis-alpha). Il en résulte que toute agression cutanée, plus particulièrement toute réaction inflammatoire et/ou irritative, capable d'induire en quantité suffisante l'une ou l'autre de ces cytokines ou les deux, est capable de stimuler la migration des CL, et donc de faciliter la réaction allergique si ces CL sont associées à un antigène.

Des pathologies de type dermatologique peuvent être observées comme résultant de la migration des CL suite à la capture d'un antigène de surface. Dans l'eczéma atopique, les CL sont capables de fixer des IgE en surface et d'induire une réponse immunitaire pathologique. Dans l'eczéma de contact, les CL jouent un rôle central puisqu'elles captent et traitent l'antigène avant de le présenter aux lymphocytes T. Celui-ci va le garder en mémoire et la réaction immunitaire sera déclenchée au deuxième contact.

Compte tenu de ce qui précède, il est hautement souhaitable de pouvoir modifier la capacité migratoire des dendrocytes dermiques, des monocytes, des lymphocytes, des cellules de Langerhans (CL), pour tenter d'augmenter le seuil de tolérance ou de limiter la réactivité de la peau allergique et/ou inflammatoire et/ou irritée et/ou âgée. C'est le problème que se propose de résoudre la présente invention. Les inventeurs ont en effet démontré de manière tout à fait surprenante et inattendue que des composés tels que les dérivés d'acide carbamique permettent d'inhiber de manière spectaculaire la migration des cellules, telles que les cellules de Langerhans, notamment la migration induite par la présence d'un agent allergène.

La présente invention a ainsi pour objet une composition comprenant au moins un dérivé d'acide carbamique de formule générale (I) : 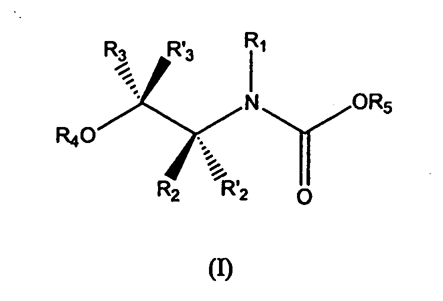 dans laquelle :
R₁ représente un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₂ et R'₂ représentent, de manière indépendante, un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₃ et R'₃ représentent, de manière indépendante, un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₄ et R₅ représentent, de manière indépendante, un atome d'hydrogène ou un groupe acyle de type RxCO, dans lequel Rx est un radical alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH).

Selon un mode de réalisation de l'invention, R₁ représente un atome d'hydrogène.

Selon un autre mode de réalisation de l'invention, R₂ représente un groupe alkyle linéaire saturé en C₈-C₂₂, avantageusement en C₉-C₁₈, de manière encore plus avantageuse en C₉-C₁₃, de manière encore plus avantageuse en C₁₁-C₁₃, et/ou R'₂ représente un atome d'hydrogène.

Selon un autre mode de réalisation de l'invention, R₃ et R'₃ représentent un atome d'hydrogène.

Selon un autre mode de réalisation de l'invention, R₄ et R₅ représentent un atome d'hydrogène.

Dans un mode de réalisation particulier de la présente invention, R₁ représente un atome d'hydrogène, R₂ représente un groupe alkyle linéaire saturé en C₈-C₂₂, avantageusement en C₉-C₁₈, de manière encore plus avantageuse en C₉-C₁₃, de manière encore plus avantageuse en C₁₁-C₁₃, et R'₂, R₃, R'₃, R₄ et R₅ représentent un atome d'hydrogène.

Avantageusement selon la présente invention, le dérivé d'acide carbamique est choisi dans le groupe constitué par le (1-Hydroxyméthyl-tridécyl)-acide carbamique et le (1-Hydroxyméthyl-undécyl)-acide carbamique.

Le (1-Hydroxyméthyl-tridécyl)-acide carbamique peut être représenté par la formule suivante :

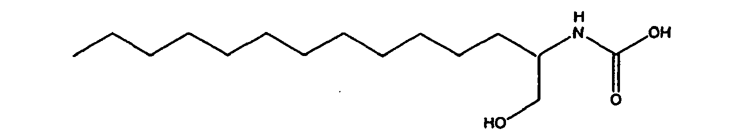

Selon un mode de réalisation de l'invention, la composition peut comprendre en outre au moins un inhibiteur de la migration des cellules, telles que les cellules de Langerhans, sélectionné dans le groupe des inhibiteurs de métalloprotéases matricielles (MMPs).

Par « composés inhibiteurs des métalloprotéases matricielles (MMPs) », on entend selon l'invention tout composé connu de l'homme du métier pour sa capacité d'inhiber l'activité de dégradation de la matrice extracellulaire par les MMPs. Les MMPs constituent une famille d'enzymes (actuellement plus d'une vingtaine ont été identifiées et caractérisées), zinc-dépendantes, de structure très conservée, qui possèdent la capacité de dégrader les composants de la matrice extracellulaire. Elles sont classées selon la nature de leur substrat en collagénases, gélatinases et stromélysines. Elles peuvent être synthétisées par différents types cellulaires au niveau de la peau (fibroblastes, kératinocytes, macrophages, cellules endothéliales, éosinophiles, cellules de Langerhans, etc.). Le groupe des MMPs est ainsi constitué de quatre sous-classes : (1) les collagénases, (2) les gélatinases, (3) les stromélysines et (4) les MMP de type membranaires (MT-MMPs). L'activité des MMPs peut être modulée par des inhibiteurs de protéinase naturellement présents tels que les inhibiteurs tissulaires de métalloprotéinases (TIMPs, notamment les TIMP-1 et TIMP-2). En particulier, le composé actif pour inhiber la migration des cellules de Langerhans est un composé inhibiteur d'au moins une MMP choisie dans le groupe constitué par les MMP-1, MMP-2, MMP-3 MMP-9, MMP-7, MMP-13 et MMP-18. Comme « composé inhibiteur des MMPs », en tant que composé actif pour inhiber la migration des cellules de Langerhans selon la présente invention, on entend en particulier les inhibiteurs tissulaires de métalloprotéinases (TIMPs), l'alpha-2-macroglobuline, les inhibiteurs de l'activateur du plasminogène, la bryostatine-1, les antibiotiques (doxycyclines, minocyclines, etc.), les peptides synthétiques ou naturels ayant une structure similaire aux substrats des MMPs (batimastat, marimastat, etc.), les rétinoïdes (en particulier les rétinoïdes non aromatiques tels que le rétinaldéhyde, la trétinoïne, et l'acide rétinoïque 9-cis, la vitamine A, les rétinoïdes monoaromatiques tels que l'étrétinate, l'all-trans acitrétine et le motrérinide, et les rétinoïdes polyaromatiques tels que l'adapalène, le tazarotène, le tamibarotène et l'arotinoïde methyl sulfone), les anti-oxydants (les piégeurs d'oxygène singulet, etc.), les anti-cancéreux (ou « anti-métastatiques »), les hydrolysats de malt tels que Colalift commercialisés par la société Coletica, les extraits d'algues marines tels que Kelpadélie commercialisés par la société Secma, les extraits de cartilage de requin tels que le complexe MDI commercialisés par la société Atrium, les peptides de riz comme par exemple Colhibin commercialisé par la société Pentapharm, et les extraits peptidiques de lupin. Plus particulièrement, le composé inhibiteur des MMPs selon la présente invention est choisi dans le groupe constitué par les extraits peptidiques de lupin ou « peptides de lupin », tels que ceux décrits dans la demande de brevet FR 99 04875 déposée le 19 avril 1999 au nom de la société Laboratoires Pharmascience. On peut notamment citer l'extrait peptidique décrit dans la demande FR 99 04875 sous la dénomination extrait B (LU105). Selon un autre mode avantageux de réalisation, ledit inhibiteur des MMPs est choisi dans le groupe constitué par les rétinoïdes.

Selon un mode de réalisation particulier de l'invention, la composition peut également comprendre en outre au moins un composé choisi dans le groupe constitué par les inhibiteurs de PKC, les agents anti-inflammatoires, les agents apaisants, les immunosuppresseurs, les chélateurs d'ions, les oxazolines et les alcanolamides. Ce ou ces composé(s) choisi(s) dans le groupe constitué par les inhibiteurs de PKC, les agents anti-inflammatoires, les agents apaisants, les immunosuppresseurs, les chélateurs de métaux, les oxazolines et les alcanolamides permet(tent) de jouer sur et/ou de limiter la réaction irritative ou de sensibilisation voire pour certains d'entre eux également d'inhiber la migration des cellules dendritiques, plus particulièrement des cellules de Langerhans, des dendrocytes dermiques, des monocytes, des lymphocytes, des kératinocytes, des mastocytes et des cellules endothéliales.

Par « PKC » ou « Protéines Kinases C », on entend au sens de la présente invention les enzymes qui catalysent une réaction de phosphorylation sur un substrat cellulaire.

Lorsqu'elles sont activées, les PKC phosphorylent des résidus sérine ou thréonine spécifiques sur des substrats protéiques, qui varient selon le type cellulaire. Dans de nombreuses cellules, l'activation des PKC augmente la transcription de gènes spécifiques.

Les protéines kinases C (PKC) sont des protéines codées par une famille de gènes (11 isoformes différentes). On sait notamment que ces protéines sont impliquées dans la transduction de signaux extracellulaires médiés par les facteurs de croissance, les cytokines, ainsi que par un certain nombre d'autres molécules biologiques. La protéine kinase β2 (PKC-β2) apparaît exprimée spécifiquement par les CL de l'épiderme.

Tout composé connu de l'homme du métier comme inhibant l'activité de phosphorylation des PKC peut ainsi être utilisé en tant que composé inhibiteur des PKC selon la présente invention. On peut par exemple citer les polypeptides décrits dans la demande WO 99/43805 (Incyte Genomics Inc.).

En particulier, le composé inhibiteur des PKC est choisi dans le groupe constitué par les inhibiteurs non spécifiques des PKC, les inhibiteurs spécifiques de l'isoforme PKC-β2, et les associations de ceux-ci.

Plus particulièrement, le composé inhibiteur des PKC est choisi dans le groupe constitué par les composés phénoliques et polyphénoliques, les procyanidines (catéchines, épicatéchines, etc.), l'alpha-amyrine, le lupéol, le lupéol linoléate, les stérols, les stanols, les alcools triterpéniques et leurs homologues hydrogénés, les antibiotiques tels que la staurosporine, Ro-318425 (ou 2-(8)-(aminométhyl)-6,7,8,9-tétrahydropyridol(1,2-a)indol-3-yl)3-(1-méthyl-indol-3-ylmaléimide, HCl) tel que commercialisé par la société Calbiochem, les composés qui agissent par compétition avec les activateurs physiologiques des PKC tels que le diacylglycérol et le phorbol ester, les lipides cutanés de type (lyso)sphingolipides, lysophospholipides tels que les céramides et pseudocéramides, sphingosines et phytosphingosines, les sphinganines, les dérivés, précurseurs, analogues et homologues de ces composés, d'origine naturelle ou synthétique.

Par « composés phénoliques et polyphénoliques », on entend selon l'invention les phénols simples, les benzoquinones, les acides phénoliques, les acétophénones, les acides phénylacétiques, les acides hydroxycinnamiques, les coumarines et isocoumarines, les chromones, les naftoquinones, les xanthones, les anthraquinones, les flavonoides, les lignanes et néolignanes, les lignines, les chalcones, les dihydrochalcones, les aurones, les flavones, les flavonols, les dihydroflavonols, les flavanones, les flavanols, les flavandiols ou leucoanthocyanidines, les anthocyanidines, les isoflavonoides, les biflavonoides, les proanthocyanidines et les tanins condensés.

Par "stérols", on entend plus particulièrement selon l'invention le stérol, c'est à dire le composé perhydro-1,2-cyclopentanophenanthrène ayant un groupe hydroxyle à la position 3, et les analogues du stérol de formule générale (I) ci-dessous.

Ainsi, de préférence, les stérols utilisables selon l'invention répondent à la formule générale suivante: 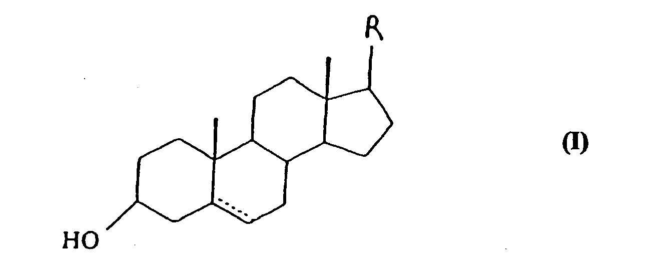 dans laquelle l'insaturation en pointillés en position 5 correspond à l'insaturation dans le cas des stérols, R représente une chaîne hydrocarbonée, linéaire ou ramifiée, insaturée ou non, comportant de 1 à 25 atomes de carbone. En particulier, R est choisi dans le groupe constitué par les groupes alkyle en C₁-C₁₂ les groupes alcoxy en C₁-C₈, les groupes alcényle en C₂-C₈, les groupe alcynyles en C₂-C₈, les groupes cycloalkyle en C₃-C₈, les groupes alcényle en C₂-C₈ halogénés, les groupes alcynyles en C₂-C₈ halogénés. Le terme "halogéné" désigne un ou plusieurs substituant halogène, à savoir un ou plusieurs atome(s) de chlore, fluor, brome ou iode.

Parmi les stérols pouvant être avantageusement utilisés selon l'invention, on peut citer en particulier le β-sitostérol, l'α-sitostérol, le γ-sitostérol, le stigmastérol, le campestérol ou encore le brassicastérol et les mélanges de ceux-ci. Par exemple, le β-sitostérol peut être utilisé sous la forme du produit dénommé "Ultra" (comprenant principalement du β-sitostérol) tel que commercialisé par la société Kaukas. Dans le cas d'une utilisation d'un mélange de stérols, on peut citer par exemple le produit dénommé "Generol" comprenant principalement du β-sitostérol (environ 50 % en poids), du stigmastérol, du brassicastérol et du campestérol tel que commercialisé par la société Cognis ou encore le produit "Primal" de la société Kaukas.

Parmi les alcools triterpéniques pouvant être avantageusement utilisés selon l'invention, on peut citer en particulier la β-amyrine, l'érythrodiol, le taraxastérol, le cycloarténol, le 24-méthylènecycloartanol, le lupéol, le lanostérol et les mélanges de ceux-ci.

Par "homologues hydrogénés" d'un alcool triterpénique, on entend selon l'invention le ou les composés alcool(s) triterpénique(s) correspondant(s) dont la ou les liaison(s) insaturée(s) éventuellement présente(s) ont été hydrogénées (c'est à dire transformée(s) en liaison saturée) selon des méthodes bien connues de l'homme du métier.

Plus particulièrement encore, le composé inhibiteur des PKC est choisi dans le groupe constitué par les sphingolipides et les lysophospholipides, comme ceux cités dans le tableau 1 suivant :

**Tableau 1 : structures de sphingolipides et lysosphingolipides**

| | SPHINGOLIPIDES | LYSOSPHINGOLIPIDES |
|---|---|---|
| Structure | 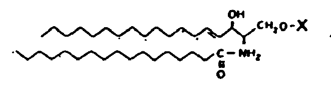 | 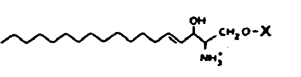 |
| X : H - | Ceramide | Sphingosine |
| X : Galactose - | Galactocerebroside | Psychosine (Galactosylsphingosine) |
| X : Sullogalactose- | Sulfatide | Lysosulforide (Sulfogalactosylsphingosine) |
| 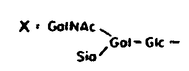 | GM₂ | Lyso GM₂ |
| X : Phosphorylcholine - | Sphingomyelin | Lysosphingomyelin |

On peut citer également plus particulièrement, comme composé inhibiteur des PKC, les lipides cutanés de type sphingolipides et lysophospholipides.

Comme sphingolipides, on peut citer ceux parmi les plus élémentaires tels que la sphingosine (D érythro dihydroxy 1,3 amino 2 octadécène 4t) et ses isomères, la phytosphingosine (D ribo trihydroxy 1,3,4 amino 2 octadécane) et ses isomères. Mais aussi, les lysosphingolipides (parmi eux, la lysosulfatide et la psychosine), la sulfogalactosylsphingosine, la sphinganine (2-amino 1,3 octadécane diol) et les sphingomyelines.

Comme phospholipides, on peut citer ceux parmi les familles des phosphatidylamino-alcools et des phosphatidylpolyols. Le groupe des phosphatidylamino-alcools comprend notamment les phosphatidylethanolamines (ou phosphatidylcolamines), les phosphatidylcholines, les phosphatidylsérines, les N-acylphosphatidylethanolamines. Celui des phosphatidylpolyols comprend quant à lui, les phosphatidylcholinositols, les diphospho-inositides, les lysodiphospho-inositides, les phosphatidylglycérols et les cardiolipides.

On peut citer également plus particulièrement, comme composé inhibiteur des PKC, les céramides, notamment les céramides du ciment intercornéocytaire de l'épiderme ainsi que les précurseurs des céramides que sont la sphingosine et la phytosphingosine.

D'une manière générale, les céramides peuvent être synthétisés chimiquement (on parle notamment de pseudo-céramides), être d'origine animale (des concentrations relativement élevées de sphingolipides sont présentes dans l'encéphale et la colonne vertébrale des mammifères), d'origine végétale (principalement des cérébrosides et autres sphingolipides glycosylées) ou encore être des dérivés de levures (configuration stéréochimique identique à celle des céramides naturellement présents dans la peau humaine).

Les céramides du ciment intercoméocytaire de l'épiderme peuvent être séparés par les méthodes classiques (chromatographie sur couche mince) en six fractions, correspondant à des composés différant par la nature des acides gras et la nature de la base impliquée (sphingosines, insaturées, ou phytosphingosines, saturées). Le tableau 2 suivant illustre les structures respectives présentes dans ces fractions, selon la classification de Werts et Downing. La fraction 6 peut elle-même être subdivisée par des méthodes plus fines en deux entités : les céramides 6a et 6b.

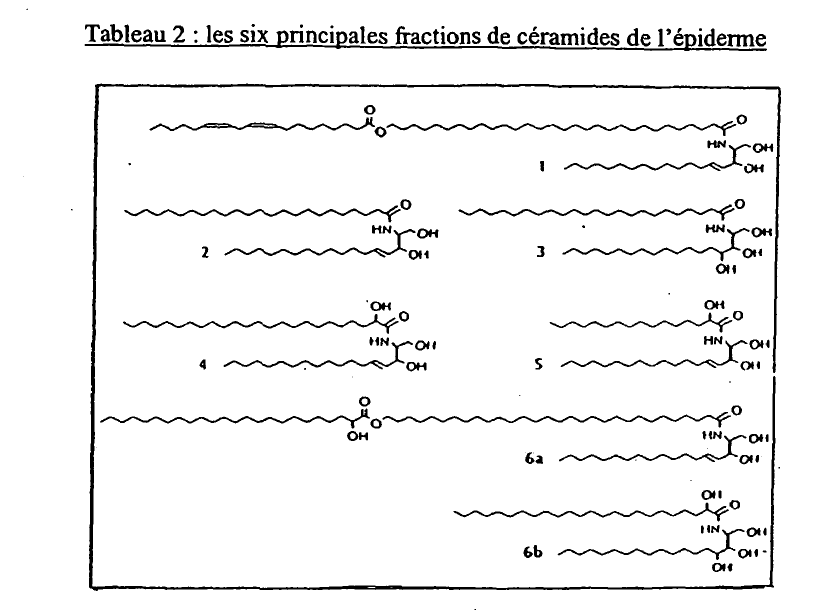

Ainsi, les céramides 1, les moins polaires, comportent une structure tout à fait particulière, que l'on retrouve dans le céramide 6a : un oméga-hydroxyacide à longue chaîne amidifiant la base, et attaché à son extrémité omega par une liaison ester à un autre acide gras (0-acylcéramides). Dans le cas de la fraction 1, les acides gras liés à la sphingosine sont essentiellement en C24, C26, C30, C32 ou C34, pouvant être saturés, monoéthyléniques (principalement pour les C30, C32 et C34) ou diéthyléniques (C32 et surtout C34). Quant à l'acide gras attaché à l'extrémité oméga du précédent, il s'agit, de façon largement prédominante pour les céramides 1, de l'acide linoléique, le rôle capital dans la fonction de barrière hybride de l'épiderme est bien connu.

La fraction 2, de structure plus classique (sphingosines ou dihydrosphingosines liées par une liaison amide à un acide gras, principalement C20 à C28), est la plus abondante.

La fraction 3 est assez similaire, la différence portant sur la nature de la base, qui, en l'occurrence, est essentiellement représentée par les phytosphingosines, saturées.

Les fractions 4 et 5 sont essentiellement caractérisées par la présence d'alpha-hydroxyacides liés à une sphingosine.

La fraction 6b est proche des fractions 4 et 5, comportant un alpha hydroxyacide, mais lié à une phytosphingosine, saturée.

La fraction 6a, comme le céramide 1, comporte le motif caractéristique que l'on ne retrouve qu'au niveau des céramides de l'épiderme, c'est-à-dire la liaison ester entre l'hydroxyle en oméga d'un acide gras lié à une sphingosine, et le groupement carboxylique d'un acide gras terminal, qui, cette fois, n'est pas l'acide linoléique, mais un alpha-hydroxyacide.

Il convient également de citer les phytocéramides (céramides à base phytosphingosine), les cholestérol-céramides synthétiques, les galacto ou gluco cérébrosides.

Enfin, parmi les composés inhibiteurs des PKC pouvant être utilisés selon la présente invention, la sphingosine est présente à l'état naturel dans la peau, et joue entre autre un rôle important dans fonction barrière du *stratum corneum,* en tant que précurseur des sphingolipides (céramides et sphingoglycolipides). Elle peut être dérivée de source biologique telle que des extraits de cerveaux bovins ou par voie de synthèse, à partir de la serine, comme décrit par exemple dans l'article de Newman, J.Am. CHEM., 95 (12) : 4098 (1973). On peut plus particulièrement citer les formes isomères de la sphingosine : D-érythro, L-thréo, L-érythro et D-thréo. La forme D-erythro est la plus fréquemment présente dans la nature.

Selon la présente invention, les composés inhibiteurs des PKC pouvant être utilisés comprennent les isomères, les dérivés (sels, complexes, etc.), les analogues, les homologues, les précurseurs et les métabolites des composés inhibiteurs des PKC décrits ci-dessus.

Les agents anti-inflammatoires pouvant être utilisés dans le cadre de la présente invention en association avec les dérivés d'acide carbamique sont avantageusement des agents anti-inflammatoires stéroïdiens (AIS), tels que les corticoïdes, ou non-stéroïdiens (AINS).

Les agents apaisants pouvant être utilisés dans le cadre de la présente invention en association avec les dérivés d'acide carbamique sont avantageusement des dérivés de réglisse et des dérivés de l'alpha-bisabolol.

Les immunosuppresseurs pouvant être utilisés dans le cadre de la présente invention en association avec les dérivés d'acide carbamique sont avantageusement le tacrolimus, le pimécrolimus, et la cyclosporine.

Les chélateurs d'ions pouvant être utilisés dans le cadre de la présente invention en association avec les dérivés d'acide carbamique sont avantageusement des chélateurs chimiques avantageusement choisis dans le groupe constitué par l'acide éthylènediamine-tétracétique (EDTA) et ses sels de sodium, de potassium, de calcium disodium, de diammonium, de triéthanololamine (TEA-EDTA), l'acide hydroxyéthyl éthylène diamine tétracétique (HEDTA) et son sel trisodique, l'acide diéthylènetriamine pentacétique (DTPA), et leurs mélanges. Les chélateurs d'ions peuvent également être des chélateurs biologiques avantageusement choisis dans le groupe constitué par la métallothionéine, la transférine, la lactoférine, la calmoduline, le chitosane méthylène phosphonate, et leurs mélanges.

Les ions chélatés sont avantageusement les ions Na⁺, K⁺, Ca²⁺, Cl⁻, Ni²⁺, Co⁺, Co²⁺, Zr ²⁺, Zr ⁴⁺, mais aussi les ions chrome au niveau d'oxydation II et III tels que Cr²⁺, Cr³⁺, et Cr₂O₇²⁻.

Les oxazolines pouvant être utilisées dans le cadre de la présente invention en association avec les dérivés d'acide carbamique sont avantageusement des oxazolines répondant aux formules générales suivantes: 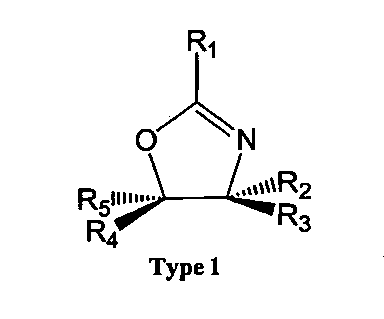 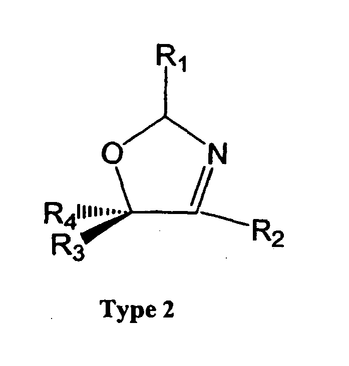 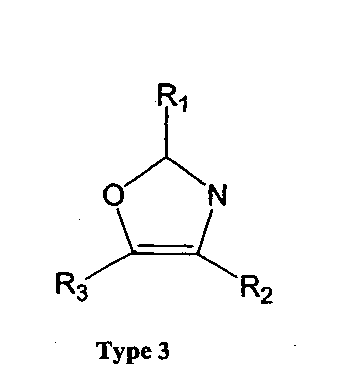 dans lesquelles R₁ représente un groupe alkyle en C₁-C₄₀, linéaire ou ramifié, saturé ou insaturé, comprenant éventuellement une ou plusieurs insaturation(s) éthylénique(s) ainsi que un ou plusieurs substituant(s) choisi(s) dans le groupe formé par les radicaux hydroxy (OH) et alcoxy en C₁-C₆ (OC₁-C₆) ; R₂, R₃, R₄ et R₅ représentent, de manière indépendante, un atome d'hydrogène, un radical hydroxy, ou un groupe alkyle en C₁-C₃₀, linéaire ou ramifié, saturé ou insaturé, comprenant éventuellement une ou plusieurs insaturations éthyléniques ainsi que un ou plusieurs substituant(s) choisi(s) dans le groupe formé par les radicaux hydroxy (OH), alcoxy en C₁-C₆ (OC₁-C₆) et alcoxy en C₁-C₆ carbonyles (COOC₁-C₆). Par le terme de "alcoxy en C₁-C₆ (OC₁-C₆)", on entend au sens de la présente invention, un radical alcoxy dont le groupement alkyle comprend de 1 à 6 atomes de carbone.

Avantageusement selon la présente invention, ladite oxazoline est une oxazoline de type 1 choisie dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline. De manière encore plus avantageuse, ladite oxazoline est la 2-undécyl-4,4-diméthyl-1,3-oxazoline, appelée OX-100, de formule :

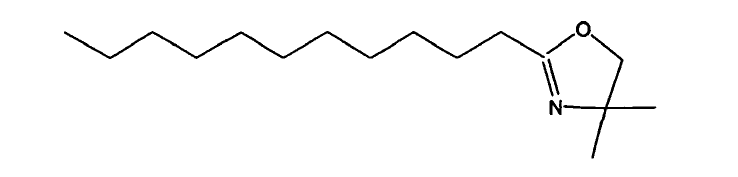

Les alcanolamides pouvant être utilisés dans le cadre de la présente invention en association avec les dérivés d'acide carbamique sont avantageusement des alcanolamides répondant à la formule générale suivante: 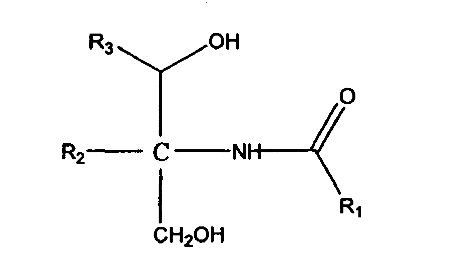 dans laquelle R₁ représente un groupe alkyle en C₁-C₄₀ comprenant de 0 à 6 insaturations et comprenant de manière éventuelle au moins un substituant choisi dans le groupe formé par les radicaux hydroxyles (OH), les alkoxy en C₁-C₆ (OC₁-C₆) et les alkoxy en C₁-C₆ carbonyles (COOC₁-C₆ ); R₂ et R₃ représentent, de manière indépendante, un atome d'hydrogène, un groupe méthyle, un groupe alkyle en C₂-C₂₀ comprenant de 0 à 6 insaturations et comprenant de manière éventuelle au moins un substituant choisi dans le groupe formé par les radicaux hydroxyles (OH), les alkoxy en C₁-C₆ (OC₁-C₆) et les alkoxy en C₁-C₆ carbonyles (COOC₁-C₆ ). Par le terme de "alcoxy en C₁-C₆ (OC₁-C₆)", on entend au sens de la présente invention, un radical alcoxy dont le groupement alkyle comprend de 1 à 6 atomes de carbone. Avantageusement selon l'invention, le radical R₁ représente un groupe alkyle comprenant de 1 à 40 atomes de carbone (en C₁-C₄₀), de manière préférée 6 à 22 atomes de carbone (en C₆-C₂₂) et de manière encore plus préférée de 8 à 18 atomes de carbone (en C₈-C₁₈), éventuellement saturé puisqu'il comprend de 0 à 6 insaturations, et comprenant de manière éventuelle au moins un radical hydroxyle, alkoxy ou alkoxycarbonyle tels que définis ci-dessus. De manière encore plus avantageuse selon la présente invention, ledit alcanolamide est l'alcanolamide appelé AK100 de formule :

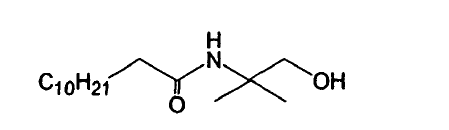

Les oxazolines et les alcanolamides selon la présente invention sont des agents inhibiteurs connus de la migration des cellules de Langerhans (Cf. demandes de brevet FR0116916et FR0116917).

Avantageusement selon la présente invention, la composition se caractérise en ce que la concentration en dérivé d'acide carbamique est comprise entre environ 0,001 et environ 10 % en poids, et plus particulièrement entre environ 0,01 et 3 % en poids, avantageusement entre 0,1 et 1 % en poids, de manière encore plus avantageuse entre 0,1 et 0,5 % en poids, par rapport au poids total de la composition.

La composition selon la présente invention est avantageusement une composition cosmétique ou pharmaceutique, notamment dermatologique. La composition selon l'invention peut être formulée sous la forme de différentes préparations adaptées à une administration topique, à une administration orale ou rectale, à une administration parentérale. De préférence, les différentes préparations sont adaptées à une administration topique et incluent les crèmes, les pommades, les lotions, les huiles, les patchs, les sprays ou tout autre produit pour application externe. Les modes d'administration, les posologies et les formes galéniques optimales des composés et compositions selon l'invention peuvent être déterminés selon les critères généralement pris en compte dans l'établissement d'un traitement cosmétique ou pharmaceutique, de préférence dermatologique, adapté à un patient comme par exemple l'âge ou le poids corporel du patient, la gravité de son état général, la tolérance au traitement, les effets secondaires constatés, et le type de peau. En fonction du type d'administration souhaité, la composition et/ou les composés actifs selon l'invention peuvent en outre comprendre au moins un excipient cosmétiquement acceptable ou pharmaceutiquement acceptable, notamment dermatologiquement acceptable. De préférence, on utilise un excipient adapté pour une administration par voie topique externe. La composition selon la présente invention peut en outre comprendre au moins un adjuvant cosmétiquement ou pharmaceutiquement connu de l'homme du métier, choisi parmi les épaississants, les conservateurs, les parfums, les colorants, les filtres chimiques ou minéraux, les agents hydratants, et les eaux thermales.

La présente invention a également pour objet les compositions décrites ci-dessus pour leur utilisation comme médicament.

La présente invention a également pour objet l'utilisation d'au moins un dérivé d'acide carbamique de formule générale (I) tel que défini ci-dessus ou d'une composition selon l'invention pour la préparation d'un médicament destiné à inhiber la migration cellules dendritiques, des dendrocytes dermiques, des monocytes, des lymphocytes, des kératinocytes, des mastocytes et des cellules endothéliales.

Selon un mode de réalisation particulier de la présente invention, le médicament est destiné à inhiber la migration des cellules de Langerhans.

Avantageusement selon la présente invention, le médicament est destiné au traitement et/ou à la prévention des réactions ou pathologies allergiques et/ou inflammatoires et/ou irritatives de la peau et/ou des muqueuses, notamment de la bouche, des poumons, de la vessie, du rectum, du vagin et des peaux âgées.

Avantageusement selon la présente invention, le médicament est destiné au traitement et/ou à la prévention des réactions ou pathologies de la peau et/ou des muqueuses consécutives à la migration des cellules, telles que les cellules de Langerhans, induites par un signal danger. On entend par « signal danger »au sens de la présente invention tout signal entraînant notamment la production de cytokines inflammatoires ou tout signal immunologique vrai du type pénétration d'un allergène.

Selon un mode de réalisation de la présente invention, le médicament est destiné au traitement et/ou à la prévention des réactions ou pathologies induites par des haptènes chimiques ou métalliques.

Selon un autre mode de réalisation de la présente invention, le médicament est destiné au traitement et/ou à la prévention des peaux et/ou des muqueuses sensibles et/ou réactives et/ou inconfortables et/ou intolérantes et/ou présentant un trouble de la barrière cutanée et/ou présentant un désordre ou un déséquilibre immunologique, tel qu'un déséquilibre clinique ou subclinique, lié au vieillissement intrinsèque, extrinsèque (soleil, pollution) ou hormonal.

En effet, il a été montré que le vieillissement de la peau entraînait une modification du statut immunitaire de celle-ci et que les cellules immunologiques pouvaient modifier leur localisation initiale par suite d'une migration incontrôlée.

La composition selon l'invention, ainsi que les composés actifs selon l'invention, permettent de réduire la réponse immunitaire induite par la migration de CL ayant fixé des IgE en surface. C'est pourquoi, la présente invention concerne également l'utilisation d'au moins un dérivé d'acide carbamique de formule générale (I) tel que défini précédemment ou d'une composition selon l'invention pour la préparation d'un médicament destiné au traitement et/ou à la prévention de l'eczéma atopique. La composition selon l'invention, ainsi que les composés actifs selon l'invention, sont également destinés au traitement et/ou à la prévention de l'eczéma de contact, dans la mesure où ils permettent de réduire une réponse immunitaire notamment induite par capture d'un antigène, traitement et présentation de cet antigène aux lymphocytes T par les CL.

La composition selon l'invention, ainsi que les composés actifs selon l'invention, sont également utilisés pour le traitement et/ou la prévention de dermatoses inflammatoires telles que le psoriasis, des dermites irritatives, des maladies auto-immunes, de la photo-immuno-suppression (diminution de la réponse immunitaire induite par les ultraviolets solaires, et plus particulièrement par les ultraviolets B), ou du rejet de greffe.

Dans les différentes utilisations précédemment évoquées du composé actif choisi dans le groupe des dérivés d'acide carbamique tels que définis ci-dessus, celui-ci peut-être utilisé en association avec au moins un composé sélectionné dans le groupe constitué par les inhibiteurs de métalloprotéases matricielles (MMPs), les inhibiteurs de PKC, les agents anti-inflammatoires, les agents apaisants, les immunosuppresseurs, les chélateurs d'ions, les oxazolines et les alcanolamides tels que définis précédemment.

La composition et les composés actifs selon l'invention sont avantageusement destinés à une utilisation en cosmétologie. La présente invention concerne également une méthode de traitement cosmétique des peaux et/ou des muqueuses sensibles, irritées, intolérantes, à tendance allergique, âgées, soumises à un signal danger, présentant un trouble de la barrière cutanée, présentant des rougeurs cutanées, ou présentant un désordre ou un déséquilibre immunologique non pathologique lié au vieillissement intrinsèque, extrinsèque ou hormonal, caractérisée en ce qu'on applique sur la peau et/ou les muqueuses au moins un dérivé d'acide carbamique de formule générale (I) tel que défini précédemment ou une composition selon l'invention. Le composé actif choisi dans le groupe des dérivés d'acide carbamique tels que définis précédemment peut également être appliqué en association avec au moins un autre composé sélectionné dans le groupe constitué par les inhibiteurs de métalloprotéases matricielles (MMPs), les inhibiteurs de PKC, les agents anti-inflammatoires, les agents apaisants, les immunosuppresseurs, les chélateurs d'ions, les oxazolines et les alcanolamides tels que définis précédemment. Dans le cadre de la méthode de traitement cosmétique selon la présente invention, le désordre ou le déséquilibre immunologique non pathologique est un déséquilibre temporaire ou non de la fonction immunitaire de la peau sans caractère de gravité.

D'autres caractéristiques et avantages de l'invention apparaissent dans la suite de la description avec l'exemple représenté ci-après. L'exemple suivant est destiné à illustrer l'invention sans aucunement en limiter la portée. A moins qu'il n'en soit précisé autrement, les pourcentages indiqués dans les exemples suivants sont des pourcentages en poids. Le (1-Hydroxyméthyl-tridécyl)-acide carbamique est dénommé ci-après composé A.

### Exemple 1 : Exemples de compositions selon la présente invention

| | % en poids |
|---|---|
| Eau | 58.1 |
| Polydécène hydrogéné | 12 |
| Glycérine | 15 |
| Carbonate de dicaprylyle | 7 |
| Glucoside de lauryle | 2,5 |
| Polyglycéryl-2 dipolyhydroxystéarate | 3,5 |
| Extrait peptidique de lupin (Protéine hydrolysée) | 0,2 |
| Acrylate/Copolymère d'acrylate d'alkyle en C₁₀₋₃₀ | 0,4 |
| Hydroxyméthylglycinate de sodium | 0,4 |
| Gomme de xanthane | 0,3 |
| Acide carbamique ou dérivé | 0,5 |
| Hydroxyde de sodium | 0,07 |
| Acide citrique | 0,03 |

### Exemple 2: Etude de l'activité du (1-Hydroxyméthyl-tridécyl)-acide carbamique sur l'inhibition de la migration des cellules dendritiques générées in vitro à partir de précurseurs CD34+ issus de sang de cordon :

### 1. Matériel et méthodes

### 1.1 Préparation des cellules de Langerhans in vitro

Des cellules mononuclées ont été obtenues à partir de sang de cordon ombilical de donneurs sains, par centrifugation sur Ficoll. Les cellules CD34+ ont ensuite été purifiées par immunosélection à l'aide d'anticorps spécifique et de billes magnétiques (Miltenyi Biotech, Allemagne). Les cellules CD34+ ont été cultivées en présence de GM-CSF (100 ng/ml), TNF-α (2,5 ng/ml) en RPMI additionné de 10% sérum de veau foetal, pendant 5 jours. L'addition de TGF-β1, facteur qui favorise la différenciation des cellules vers la voie cellules de Langerhans, a été réalisée au cinquième jour de culture.

### 1.2 Préparation des milieux

Le milieu de base choisi pour l'ensemble de l'étude a été le RPMI 1640 (Gibco BRL, France). Le composé A fourni par EXPANSCIENCE, à la concentration de 10⁻² M en solution dans du DMSO (Diméthyl Sulfoxide), a été dilué en RPMI-1640 et testé à 1 µM.

### 1.3 Sensibilisation des CL

Les cellules ont été traitées, au septième jour, par l'haptène BB (Base de Brandowski, 1,17 µg/ml), pendant 24 h, puis soumises au test de migration.

### 1.4 Migration des CL

Un système de chambre de culture à deux compartiments (Falcon, Becton Dickinson, France) a été utilisé. Le compartiment supérieur est séparé du compartiment inférieur par une membrane de porosité 8 µm, sur laquelle sont déposées 50 µg/cm² de Matrigel. La membrane est alors recouverte de protéines formant un film équivalent à une membrane basale (laminine, collagène IV, nidogène, entactine, héparane sulfate protéoglycanes). Les cellules reprises dans le milieu RPMI-BSA seul ou en présence des différents produits sont déposées dans le compartiment supérieur. Dans le compartiment inférieur, est ajouté du surnageant de culture de fibroblastes humains normaux. Après 18h d'incubation à 37°C, le nombre de cellules vivantes ayant traversé le Matrigel et se trouvant dans le compartiment inférieur est compté sous microscope (les CL sont facilement identifiables par leur forme dendritique). Chaque essai est réalisé en triplicate.

### 2. Résultats

Les résultats portant sur la migration des CL sont présentés dans le tableau 3 suivant.

**Tableau 3 :**

| | Cellules sensibilisées par l'haptène BB | BB + Composé A (1 µM) |
|---|---|---|
| Pourcentage de cellules dendritiques générées *in vitro* ayant migré | 20 | 15 |

Le tableau 3 montre ainsi le pourcentage de cellules ayant migré en présence du produit testé. Le pourcentage est calculé en rapportant le nombre de cellules récupérées dans le compartiment inférieur de la chambre de migration au nombre de cellules soumises à la migration.

Ainsi, le composé A à la concentration de 1 µM inhibe de manière significative (25 %) la migration des cellules dendritiques générées *in vitro* et activées par l'haptène BB.

En conclusion, il a ainsi été montré, de manière tout à fait surprenante, qu'en utilisant des cellules dendritiques générées *in vitro* et activées par l'haptène BB, placées dans un système de chambre de culture à deux compartiments (permettant la migration cellulaire), le (1-Hydroxyméthyl-tridécyl)-acide carbamique inhibe de manière significative la migration des cellules dendritiques générées *in vitro.*

### Exemple 3 : Etude de l'activité du (1-Hydroxyméthyl-tridécyl)-acide carbamique sur l'inhibition de la migration des cellules dendritiques sur la souris

### 1. Matériels & méthodes

### 1.1 Réactifs

Le FITC (Fluorescéine isothiocyanate, Sigma, St. Louis, MO) a été dilué extemporanément dans un mélange acétone: dibutylphthalate (1:1).

### 1.2 Inhibiteurs

L'acide carbamique A (acide 1-Hydroxyméthyl-tridécyl carbamique) et le LU105 (LU 105 est un inhibiteur de MMPs, correspondant à un extrait peptidique de lupin blanc commercialisé par la Société Expanscience sous le nom de marque Actimp 193®) ont été fournis par les "Laboratoires Expanscience", et formulés seuls ou en association dans un véhicule inerte compatible avec une application topique tel que décrit à l'exemple 1 ci-dessus [Acide carbamique A (0,5%) ± LU105 (2%)]. Les différentes formulations ont été appliquées sur les oreilles de souris deux fois par jour pendant 4 jours consécutifs. Trois heures après la dernière application, 1,5% de FITC sont appliqués sur les deux oreilles (l'une traitée et l'autre non traitée (Contrôle)).

### 1.3 Migration des cellules de Langerhans (CL) et des cellules dendritiques (CD) sur

### la souris

L'effet des deux molécules a été évalué in vivo chez la souris. La peau des deux oreilles est badigeonnée avec 1,5% FITC (2X5 µl). 24 h après, les souris sont sacrifiées et une suspension cellulaire est préparée à partir des ganglions auriculaires et cervicaux (ganglions drainants, ci-après dénommés GL) ou à partir des ganglions popliteaux (ganglions non drainants, contrôle négatif). Les tissus ont été découpés et les cellules sont séparées par filtration (filtre 100 µM, Falcon; Becton Dickinson), puis lavés. Les cellules sont ensuite centrifugées 10 min à 600 x g (m x s⁻²) sur gradient de metrizamide (14,5% dans du RPMI 1640 ; 7,5% SVF). Les cellules de l'interface sont récupérées, rincées puis marquées avec un AC anti-CDS86 PE-conjugué, biot-MHC CLII mAbs plus streptavidine-Cya (PharMingen) et analysées par cytométrie en flux. Seules les cellules FITC+, PE+ et Cya+ sont comptabilisées car elles représentent la population de cellules ayant migré de la peau vers les GL suite à l'application de l'haptène.

### 2. Résultats

L'application topique du véhicule seul n'entraîne aucune modification du nombre de CD FITC+ au niveau des GL. Le véhicule est donc sans effet sur les capacités migratoire des CD.

Les résultats portant sur la migration des CD sont présentés dans le tableau 4 suivant.

**Tableau 4 :**

| | Acide carbamique A (1) (0,5%) | LU105 (2%) | Acide carbamique A (1) (0,5%) + LU105 (2%) |
|---|---|---|---|
| Inhibition de la migration en % | 25 | 30 | 85 |

| | | | |
|---|---|---|---|
| (1) acide 1-Hydroxyméthyl-tridécyl carbamique | | | |

La migration des CD au niveau des GL, suite à l'application de l'haptène FITC, est inhibée dans des proportions comparables et sans différence significative par le composé A à la dose de 0,5% et le LU105 à la dose de 2%.

Lorsque les deux types de molécules sont associés, cette inhibition est presque totale. En conclusion, il a ainsi été montré de manière tout à fait surprenante, en utilisant un modèle de souris sensibilisées par l'haptène FITC, que l'acide 1-Hydroxyméthyl-tridécyl carbamique inhibe de manière significative la migration des CD vers les GL. Par ailleurs, l'acide 1-Hydroxyméthyl-tridécyl carbamique et le LU105 agissent en synergie pour inhiber la migration des CD chez la souris sensibilisée.

## Revendications

1. Composition comprenant au moins un dérivé d'acide carbamique de formule générale (I) : 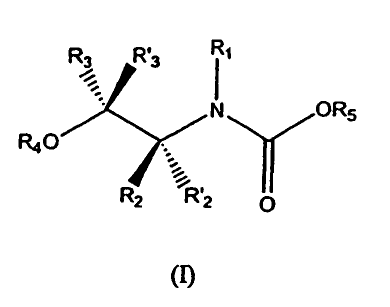 dans laquelle :
R₁ représente un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₂ et R'₂ représentent, de manière indépendante, un atome d'hydrogène ou un groupe alkyle en C₁-C₃₀, linéaire ou ratifié, saturé ou insaturé, comprenant éventuellement une ou plusieurs insaturation(s) éthylénique(s) et/ou acétylénique(s), ainsi que un ou plusieurs substituant(s) hydroxy (OH);
R₃ et R'₃ représentent, de manière indépendante, un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₄ et R₅ représentent, de manière indépendante, un atome d'hydrogène ou un groupe acyle de type RxCO, dans lequel Rx est un radical alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH).

2. Composition selon la revendication 1, **caractérisée en ce que** R₁ représente un atome d'hydrogène.

3. Composition selon la revendication 1 ou 2, **caractérisée en ce que** R₂ représente un groupe alkyle linéaire saturé en C₈-C₂₂, avantageusement en C₉-C₁₈, de manière encore plus avantageuse en C₉-C₁₃, et R'₂ représente un atome d'hydrogène.

4. Composition selon l'une quelconque des revendications 1 à 3, **caractérisée en ce que** R₃ et R'₃ représentent un atome d'hydrogène.

5. Composition selon l'une quelconque des revendications 1 à 4, **caractérisée en ce que** R₄ et R₅ représentent un atome d'hydrogène.

6. Composition selon l'une quelconque des revendications 1 à 5, **caractérisée en ce que** ledit dérivé d'acide carbamique est choisi dans le groupe constitué par le (1-Hydroxyméthyl-tridécyl)-acide carbamique et le (1-Hydroxyméthyl-undécyl)-acide carbamique.

7. Composition selon l'une quelconque des revendications précédentes, **caractérisée en ce qu'**elle contient en outre au moins un inhibiteur de métalloprotéases matricielles (MMPs), avantageusement sélectionné dans le groupe constitué par les inhibiteurs tissulaires de métalloprotéinases, l'alpha-2-macroglobuline, les inhibiteurs de l'activateur du plasminogène, la bryostatine-1, les antibiotiques, les peptides synthétiques ou naturels ayant une structure similaire aux substrats des MMPs, les rétinoïdes, les anti-oxydants, les anti-cancéreux, les hydrolysats de malt, les extraits d'algues marines, les extraits de cartilage de requin et les extraits peptidiques de lupin tels que l'extrait B (LU105).

8. Composition selon l'une quelconque des revendications précédentes, **caractérisée en ce qu'**elle contient en outre au moins un composé choisi dans le groupe constitué par les inhibiteurs de PKC, les agents anti-inflammatoires, les agents apaisants, les immunosuppresseurs, les chélateurs d'ions, les oxazolines et les alcanolamides,

9. Composition selon l'une quelconque des revendications précédentes, **caractérisée en ce que** la concentration en dérivé d'acide carbamique est comprise entre environ 0,001 et environ 10 % en poids, avantageusement entre 0,1 et 1 % en poids, par rapport au poids total de la composition.

10. Composition selon l'une quelconque des revendications précédentes pour son utilisation comme médicament.

11. Utilisation d'une composition selon l'une quelconque des revendications 1 à 9 pour la préparation d'un médicament.

12. Utilisation selon la revendication 11, **caractérisée en ce que** le médicament est destiné au traitement ou à la prévention des réactions ou pathologies allergiques et/ou inflammatoires et/ou irritatives **de la peau et/ou des muqueuses**.

13. Utilisation selon la revendication 11, **caractérisée en ce que** le médicament est destiné au traitement ou à la prévention des réactions ou pathologies induites par des haptènes chimiques ou métalliques.

14. Utilisation selon la revendication 11, **caractérisée en ce que** le médicament est destiné au traitement ou à la prévention des peaux et/ou des muqueuses sensibles et/ou réactives et/ou inconfortables et/ou intolérantes et/ou présentant un trouble de la barrière cutanée et/ou présentant un déséquilibre immunologique lié au vieillissement intrinsèque, extrinsèque ou hormonal.

15. Utilisation selon la revendication 11, **caractérisée en ce que** le médicament est destiné au traitement ou à la prévention de l'eczéma atopique et/ou de contact, des dermatoses inflammatoires telles que le psoriasis, des dermites irritatives, des maladies auto-immunes, de la photo-immunosuppression, ou du rejet de greffe.

16. Dérivé d'acide carbamique de formule générale (I) tel que défini à l'une quelconque des revendications 1 à 6 ou une composition selon l'une quelconque des revendications 1 à 9, appliqué sur la peau et/ou les muqueuses, pour le traitement cosmétique des peaux et/ou des muqueuses sensibles irritées, intolérantes, à tendance allergique, âgées, présentant un trouble de la barrière cutanée, présentant des rougeurs cutanées, ou présentant un déséquilibre immunologique non pathologique lié au vieillissement intrinsèque, extrinsèque en hormonal.

## Claims

1. Composition comprising at least one carbamic acid derivative according to the general formula (I): 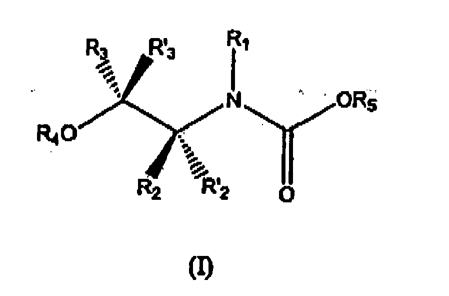 wherein:
R₁ represents a hydrogen atom or an alkyl group in C₁-C₃₀, that is linear or branched, saturated or unsaturated, possibly comprising one or more ethylene and/or acetylene insaturations, and one or more hydroxy (OH) substituents;
R₂ and R'₂ represent, independently, a hydrogen atom or an alkyl group in C₁-C₃₀, that is linear or branched, saturated or unsaturated, possibly comprising one or more ethylene and/or acetylene insaturations, and one or more hydroxy (OH) substituents;
R₃ and R'₃ represent, independently, a hydrogen atom or an alkyl group in C₁-C₃₀, that is linear or branched, saturated or unsaturated, possibly comprising one or more ethylene and/or acetylene insaturations, and one or more hydroxy (OH) substituents;
R₄ and R₅ represent, independently, a hydrogen atom or an RxCO type acyl group, wherein Rx is an alkyl radical in C₁-C₃₀, that is linear or branched, saturated or unsaturated, possibly comprising one or more ethylene and/or acetylene insaturations, and one or more hydroxy (OH) substituents.

2. Composition according to claim 1, **characterised in that** R₁ represents a hydrogen atom.

3. Composition according to claim 1 or 2, **characterised in that** R₂ represents a saturated linear alkyl group in C₈-C₂₂, advantageously in C₉-C₁₈, more advantageously in C₉-C₁₃, and R'₂ represents a hydrogen atom.

4. Composition according to any of claims 1 to 3, **characterised in that** R₃ and R'₃ represent a hydrogen atom.

5. Composition according to any of claims 1 to 4, **characterised in that** R₄ and R₅ represent a hydrogen atom.

6. Composition according to any of claims 1 to 5, **characterised in that** said carbamic acid derivative is selected from the group consisting of (1-Hydroxymethyl-tridecyl)-carbamic acid and (1-Hydroxymethyl-undecyl)-carbamic acid.

7. Composition according to any of the preceeding claims, **characterised in that** it also contains at least one matrix metalloprotease (MMP) inhibitor, advantageously selected from the group consisting of metalloprotease tissue inhibitors, alpha-2-macroglobulin, plasminogen activator inhibitors, bryostatin-1, antibiotics, synthetic or natural peptides having a similar structure to MMP substrates, retinoids, antioxidants, anticancer agents, malt hydrolysates, marine algae extracts, shark cartilage extracts and rabbit peptide extracts such as extract B (LU105) .

8. Composition according to any of the preceeding claims, **characterised in that** it also contains at least one compound selected from the group consisting of PKC inhibitors, anti-inflammatory agents, soothing agents, ion chelators, oxazolines and alcanolamides.

9. Composition according to any of the preceeding claims, **characterised in that** the carbamic acid derivative concentration is between approximately 0.001 and approximately 10% by weight, advantageously between 0.1 and 1% by weight, with respect to the total weight of the composition.

10. Composition according to any of the preceeding claims for the use thereof as a drug.

11. Use of a composition according to any of claims 1 to 9 for the preparation of a drug.

12. Use according to claim 11, **characterised in that** the drug is intended for the treatment or prevention of allergic and/or inflammatory and/or irritant reactions or conditions of the skin and/or mucosa.

13. Use according to claim 11, **characterised in that** the drug is intended for the treatment and prevention of reactions or conditions induced by chemical or metallic haptenes.

14. Use according to claim 11, **characterised in that** the drug is intended for the treatment or prevention of skins and/or mucosa that are sensitive and/or reactive and/or subject to discomfort and/or intolerant and/or display a skin barrier disorder and/or display an immunological imbalance associated with intrinsic, extrinsic or hormonal aging.

15. Use according to claim 11, **characterised in that** the drug is intended for the treatment or prevention of atopic and/or contact eczema, inflammatory dermatosis such as psoriasis, irritative dermitis, autoimmune diseases, photo-immunosuppression, or graft rejection.

16. Carbamic acid derivative according to general formula (I) as defined in any of claims 1 to 6 or a composition according to any of claims 1 to 9, applied to the skin and/or mucosa, for the cosmetic treatment of skins and/or mucosa that are sensitive, irritated, intolerant, with allergic tendencies, aged, display a skin barrier disorder, display skin rash, or display a non-pathological immunological imbalance associated with intrinsic, extrinsic or hormonal aging.

## Patentansprüche

1. Verbindung, umfassend mindestes ein Derivat der Karbamidsäure mit der allgemeinen Formel (I): 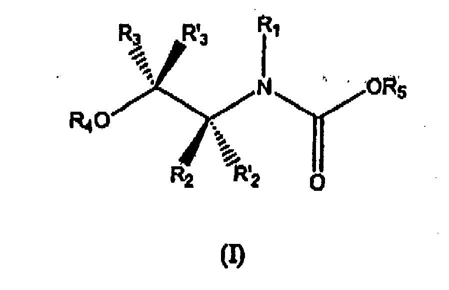 in der:
R₁ ein Wasserstoffatom oder eine C₁-C₃₀-Alkylgruppe, unverzweigt oder verzweigt, gesättigt oder ungesättigt, darstellt, umfassend eventuell eine oder mehrere ethylenische und / oder azetylenische ungesättigte Verbindungen, ebenso wie ein oder mehrere Hydoxy-Substituenten (OH);
R₂ und R'₂ auf unabhängige Weise ein Wasserstoffatom oder eine C₁-C₃₀-Alkylgruppe, unverzweigt oder verzweigt, gesättigt oder ungesättigt, darstellen, umfassend eventuell eine oder mehrere ethylenische und / oder azetylenische ungesättigte Verbindungen, ebenso wie ein oder mehrere Hydoxy-Substituenten (OH);
R₃ und R'₃ auf unabhängige Weise ein Wasserstoffatom oder eine C₁-C₃₀-Alkylgruppe, unverzweigt oder verzweigt, gesättigt oder ungesättigt, darstellen, umfassend eventuell eine oder mehrere ethylenische und / oder azetylenische ungesättigte Verbindungen, ebenso wie ein oder mehrere Hydoxy-Substituenten (OH);
R₄ und R₅ auf unabhängige Weise ein Wasserstoffatom oder eine Acylgruppe des Typs RxCO, in der Rx ein C₁-C₃₀-Alkylradikal ist, unverzweigt oder verzweigt, gesättigt oder ungesättigt, darstellen, umfassend eventuell eine oder mehrere ethylenische und / oder azetylenische ungesättigte Verbindungen, ebenso wie ein oder mehrere Hydoxy-Substituenten (OH).

2. Verbindung nach Anspruch 1, **dadurch gekennzeichnet, dass** R₁ ein Wasserstoffatom darstellt.

3. Verbindung nach Anspruch 1 oder 2, **dadurch gekennzeichnet, dass** R₂ eine unverzweigte, gesättigte C₈-C₂₂-, vorteilhafterweise C₉-C₁₈-, noch besser C₉-C₁₃-Alkylgruppe und R'₂ ein Wasserstoffatom darstellt.

4. Verbindung nach einem der Ansprüche 1 bis 3, **dadurch gekennzeichnet, dass** R₃ und R'₃ ein Wasserstoffatom darstellen.

5. Verbindung nach einem der Ansprüche 1 bis 4, **dadurch gekennzeichnet, dass** R₄ und R₅ ein Wasserstoffatom darstellen.

6. Verbindung nach einem der Ansprüche 1 bis 5, **dadurch gekennzeichnet, dass** das Derivat von Karbamidsäure aus der Gruppe ausgewählt wird, die aus der (1-Hydroxymethyl-Tridecyl)-Karbamidsäure und der (1-Hydroxymethyl-Undecyl)-Karbamidsäure besteht.

7. Verbindung nach einem der vorhergehenden Ansprüche, **dadurch gekennzeichnet, dass** sie außerdem mindestens einen Inhibitor der Matrix-Metalloproteasen (MMPs) enthält, vorzugsweise ausgewählt aus der Gruppe, die aus den Gewebeinhibitoren der Metalloproteinasen, dem Alpha-2-Makroglobulin, den Inhibitoren des Aktivators des Plasminogens, dem Bryostatin-1, den Antibiotika, den synthetischen oder natürlichen Peptiden, die eine ähnliche Struktur wie die Substrate der MMPs aufweisen, den Retinoiden, den Antioxidationsmitteln, den Antikrebsmitteln, den Malzhydrolysaten, den Extrakten von Meeresalgen, den Extrakten von Haiknorpeln und den Peptidextrakten der Lupine wie z.B. dem Extrakt B (LU105) besteht.

8. Verbindung nach einem der vorhergehenden Ansprüche, **dadurch gekennzeichnet, dass** sie außerdem mindestens eine Zusammensetzung umfasst, die aus der Gruppe gewählt ist, die aus den Inhibitoren des PKC, den Entzündungshemmern, den beruhigenden Mitteln, den immunsuppressiven Mitteln, den Ionen-Chelatbildnern, den Oxazolinen und den Alkanolamiden besteht.

9. Verbindung nach einem der vorhergehenden Ansprüche, **dadurch gekennzeichnet, dass** die Konzentration des Derivats der Karbamidsäure zwischen ungefähr 0,001 und ungefähr 10 Gew.-%, vorteilhafterweise zwischen 0,1 und 1,0 Gew.-% bezüglich des Gesamtgewichtes der Verbindung liegt.

10. Verbindung nach einem der vorhergehenden Ansprüche zur Verwendung als Medikament.

11. Verwendung einer Verbindung nach einem der vorhergehenden Ansprüche 1 bis 9 für die Zubereitung eines Medikaments.

12. Verwendung nach Anspruch 11, **dadurch gekennzeichnet, dass** das Medikament für die Behandlung oder die Prävention von allergischen und / oder entzündlichen und / oder reizenden Reaktion oder Pathologien der Haut und / oder der Schleimhäute vorgesehen ist.

13. Verwendung nach Anspruch 11, **dadurch gekennzeichnet, dass** das Medikament für die Behandlung oder die Prävention von Reaktionen oder Pathologien vorgesehen ist, die von chemischen oder metallischen Haptenen verursacht werden.

14. Verwendung nach Anspruch 11, **dadurch gekennzeichnet, dass** das Medikament für die Behandlung oder die Prävention von sensibler und / oder reaktiver und / oder unangenehmer und / oder unverträglicher und / oder ein Problem der Hautbarriere aufweisender und /oder ein immunologisches Ungleichgewicht in Verbindung mit der intrinsischen, extrinsischen oder hormonalen Alterung aufweisender Haut oder Schleimhaut vorgesehen ist.

15. Verwendung nach Anspruch 11, **dadurch gekennzeichnet, dass** das Medikament für die Behandlung des endogenen Ekzems und / oder des Kontaktekzems, der entzündlichen Dermatosen wie z.B. der Psoriasis, der reizenden Dermatiten, der Auto-Immunkrankheiten, der Foto-Immunosuppression oder der Abstoßung eines Transplantates vorgesehen ist.

16. Derivat der Karbamidsäure mit einer allgemeinen Formel (I) wie in einem der Ansprüche 1 bis 6 definiert oder Verbindung nach einem der Ansprüche 1 bis 9, angewandt auf die Haut und / oder die Schleimhäute, zur kosmetischen Behandlung der sensiblen, gereizten, intoleranten, zu Allergien tendierenden, gealterten, ein Problem der Hautbarriere aufweisenden, Hautrötungen aufweisenden oder ein immunologisches, nicht pathologisches, mit der intrinsischen, extrinsischen oder hormonalen Alterung verbundenes Ungleichgewicht aufweisenden Haut und / oder Schleimhaut.
